Europäisches Patentamt

European Patent Office

Office européen des brevets

(11) Numéro de publication: **0 347 353**
**A1**

## (12) DEMANDE DE BREVET EUROPEEN

(21) Numéro de dépôt: 89440056.3

(22) Date de dépôt: 15.06.89

(51) Int. Cl.4: **A 61 N 1/39**

(30) Priorité: 15.06.88 FR 8808204

(43) Date de publication de la demande:
20.12.89 Bulletin 89/51

(84) Etats contractants désignés:
AT BE CH DE ES GB GR IT LI LU NL SE

(71) Demandeur: ATESYS, société anonyme
44, rue de l'Industrie
F-67160 Wissembourg (FR)

(72) Inventeur: Cansell, Albert
7, rue du Berger Altenstadt
F-67160 Wissembourg (FR)

(74) Mandataire: Metz, Paul
Cabinet METZ PATNI 63, rue de la Ganzau
F-67100 Strasbourg (FR)

(54) Défibrillateur de hautes performances à plusieurs électrodes extérieures au coeur.

(57) Défibrillateur de hautes performances .
Défibrillateur caractérisé en ce qu'il comporte au moins une électrode de décharge (10) située dans le ventricule droit et plusieurs électrodes extérieures au coeur, (16 à 20) éloignées de celui-ci dans des directions différentes.
Cette invention intéresse les constructeurs de défibrillateurs implantables.

FIG.1

EP 0 347 353 A1

## Description

## Défibrillateur de hautes performances à plusieurs électrodes extérieures au coeur.

La présente invention se rapporte à un défibrillateur de hautes performances comportant une électrode intérieure et plusieurs électrodes extérieures au coeur.

Diverses anomalies de fonctionnement du coeur aboutissent à un battement irrégulier et, dans les cas extrêmes, à un arrêt du pompage du sang par mise en fibrillation ventriculaire entraînant la mort.

Un défibrillateur a pour but, grâce à un choc électrique approprié, d'intensité suffisante, provenant de la décharge rapide d'un condensateur, d'exciter et de dépolariser en même temps les cellules de la plus grande masse possible du myocarde ventriculaire réduisant la fibrillation et aboutissant à la reprise des contractions rythmiques de fonctionnement normal du coeur.

A un degré de gravité inférieur, ce genre de trouble est appelé tachycardie ventriculaire. Ce trouble peut également être traité par un choc électrique. Le traitement est alors appelé cardioversion et l'appareil possédant cette fonction cardioverteur.

Dans ce qui suit, les termes de défibrillation et de défibrillateur couvrent la fonction de cardioversion et l'appareil qui la met en oeuvre.

Un défibrillateur automatique délivre automatiquement ce choc électrique dès la détection d'un trouble du rythme ou d'un arrêt tels qu'indiqués ci-dessus.

La recherche de miniaturisation est un souci constant dans la conception des dispositifs implantés dans le corps humain. Cette exigence joue encore plus dans le cadre d'un système complexe et automatique tel qu'un défibrillateur automatique.

En effet, la fonction de défibrillation et, corrélativement, la fonction de cardioversion nécessitent au moins deux électrodes entre lesquelles s'effectue la décharge, et de préférence au moins une électrode supplémentaire pour la détection du trouble du rythme et le contrôle de la décharge. Elle nécessite aussi les circuits appropriés y relatifs.

Par ailleurs, la gestion du fonctionnement automatique implique l'existence de circuits adaptés qui viennent grossir la taille de l'unique boîtier renfermant l'ensemble des circuits d'alimentation, de surveillance et de commande.

De plus, le condensateur, en raison de sa forte capacité, occupe une place relativement importante.

Ne pouvant miniaturiser plus les circuits et la pile dans l'état actuel de la technologie, les efforts doivent se reporter sur le condensateur.

De gros progrès technologiques et mises au point effectués par le déposant ont permis, par de nouvelles matières, un bobinage adapté et un savoir-faire étendu, de réduire notablement la taille des condensateurs à capacité égale.

Une des possibilités de diminution de capacité de tels condensateurs et donc de leur taille serait l'augmentation de la tension de charge. Or, le caractère de nocivité observé sur le myocarde des forts potentiels et courants locaux en résultant, ne permet pas d'envisager cette solution.

Un autre objectif, apparu récemment concernant les défibrillateurs implantables automatiques, consiste à viser un mode d'implantation ne nécessitant pas de thoracotomie.

La présente invention se fixe comme but général de remplir simultanément les deux exigences ci-dessus qui apparaissent contradictoires pour la raison suivante.

Les modes d'implantation connus à ce jour ne nécessitant pas de thoracotomie entraînent inévitablement une perte d'énergie qui doit être compensée par de meilleures performances de l'appareil déjà technolo giquement limité en poids et en taille.

Ces meilleures performances sont représentées par les améliorations de gain en poids et en encombrement et une configuration ou structure générale appropriée permettant de diminuer notablement l'énergie efficace de défibrillation et partant de cardioversion.

Dans le sentiment d'être arrivés à une limite technologique, les chercheurs du déposant se sont tournés vers les possibilités de diminuer le seuil d'énergie de la décharge correspondant à un taux d'efficacité donné.

On sait qu'à un taux d'efficacité donné, la décharge doit dépasser un seuil d'énergie minimale pour provoquer la défibrillation.

Si la valeur de ce seuil dépend des caractéristiques physiques de la personne, il dépend aussi d'autres paramètres et, dans une plus large mesure, d'un nombre restreint de facteurs mis en évidence, influant notablement et directement sur l'efficacité du système d'électrodes dans l'obtention d'un meilleur résultat statistique de défibrillation.

Il s'agit des deux facteurs importants suivants :
. le rendement énergétique de défibrillation caractérisé par le rapport entre, l'énergie appliqué au myocarde et l'énergie totale délivrée par le condensateur ;
. l'homogénéité de la densité de courant à travers le myocarde.

Ces deux facteurs dépendent eux-mêmes de nombreux paramètres et sont liés directement à la taille du condensateur proportionnelle elle-même à l'énergie qu'il peut emmagasiner.

Dans cet esprit et à la lumière des résultats provenant des expérimentations effectuées sur les animaux, ces facteurs doivent pourvoir être sensiblement améliorés.

Ces expérimentations ont permis également d'inventorier le nombre de paramètres dont ces facteurs dépendent et l'importance relative de certains d'entre eux dans le résultat final d'efficacité statistique de défibrillation.

Les défibrillateurs automatiques implantables les plus performants actuellement permettent de descendre le seuil d'énergie d'une décharge de défibrillation à une valeur d'environ 25 Joules sous une tension comprise entre 1500 et 2500 volts avec un taux d'efficacité de 95 %.

La présente invention a pour but d'améliorer sensiblement ces performances en augmentant d'une part le taux d'efficacité pour le seuil d'énergie indiqué ci-dessus et en diminuant sensiblement d'autre part le seuil d'énergie à un taux d'efficacité donné.

La présente invention a pour but de fournir les moyens nécessaires à l'obtention d'un résultat optimal de défibrillation, en tenant compte des impératifs divers liés à l'environnement et aux différentes techniques chirurgicales d'implantation, de manière à obtenir un rapport énergétique de défibrillation le meilleur possible.

A cet effet, l'invention se rapporte à un défibrillateur de hautes performances, caractérisé en ce qu'il comporte au moins une électrode de décharge intérieure au coeur, située dans le ventricule droit et plusieurs électrodes extérieures éloignées du coeur dans plusieurs directions différentes, électrodes extérieures reliées électriquement entre elles, l'ensemble des électrodes étant relié au circuit correspondant d'un boîtier d'alimentation et de commande, électrodes extérieures placées pour que la densité de courant à travers le myocarde ventriculaire se répartisse de façon homogène par une multitude de lignes de courant traversant préférentiellement les zones les plus épaisses du myocarde représentant l'essentiel de la masse myocardique en vue d'obtenir un seuil de défibrillation et/ou de cardioversion minimal.

Une caractéristique supplémentaire concerne le maintien dans le ventricule droit de l'électrode de décharge en position écartée de chacune des parois.

Une caractéristique supplémentaire de l'invention concerne sa particulière aptitude à fonctionner à des courants forts résultant de tensions de charge plus élevées sans présenter le moindre caractère de nocivité détectable, grâce à la pluralité, à l'éloignement et à l'étendue des électrodes qui permettent de réaliser la répartition multiple des lignes efficaces de courant à travers le myocarde traduisant le degré d'homogénéité de la distribution du courant à travers le coeur.

Une autre caractéristique supplémentaire concerne la mesure du courant et une possibilité de son réglage dans chacune des liaisons électriques entre le boîtier et les électrodes implantées extérieurement au coeur.

Une dernière caractéristique supplémentaire concerne l'évaluation des performances du système d'électrodes et de sa configuration optimale sur un sujet donné par application de la technique de stimulation.

La présente invention sera bien comprise à la lecture de la description qui suit, effectuée à titre d'exemple non limitatif sur plusieurs modes d'exécution et variantes en référence aux dessins accompagnants dans lesquels :

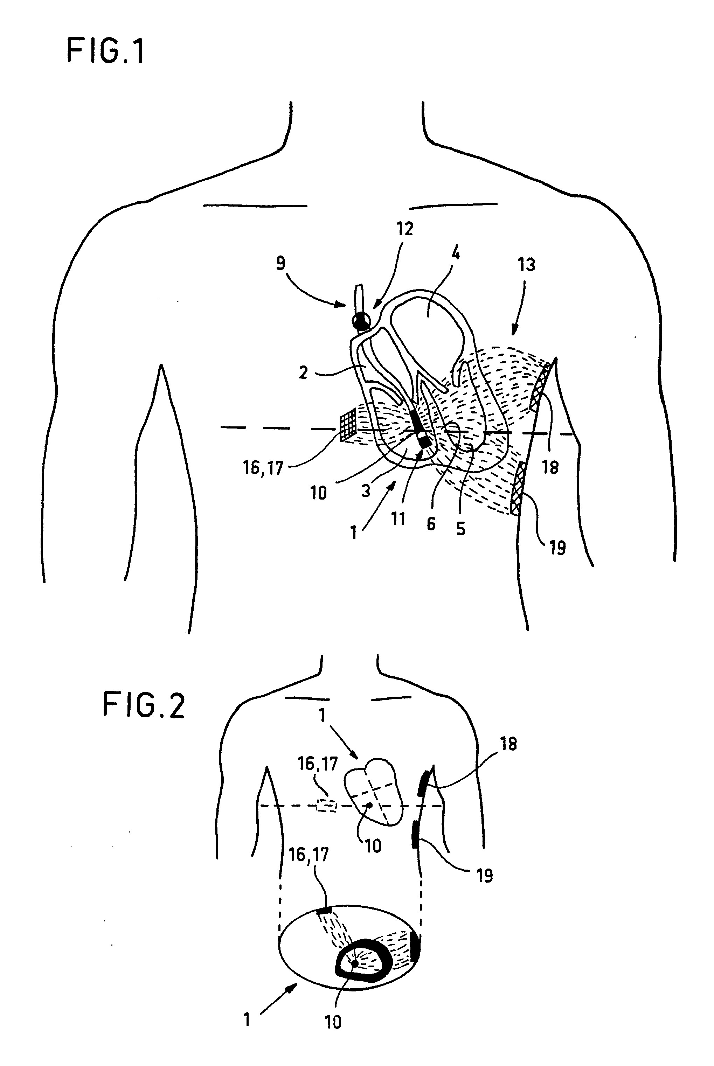

. la figure 1 est une vue générale mixte frontale en perspective et en coupe illustrant en transparence le principe de base de l'invention sans figuration des liaisons électriques ;

. la figure 2 est une vue double frontale en transparence et en coupe selon un plan transversal au coeur avec figuration des emplacements des électrodes extérieures correspondant à la variante représentée sur la figure 1 ;

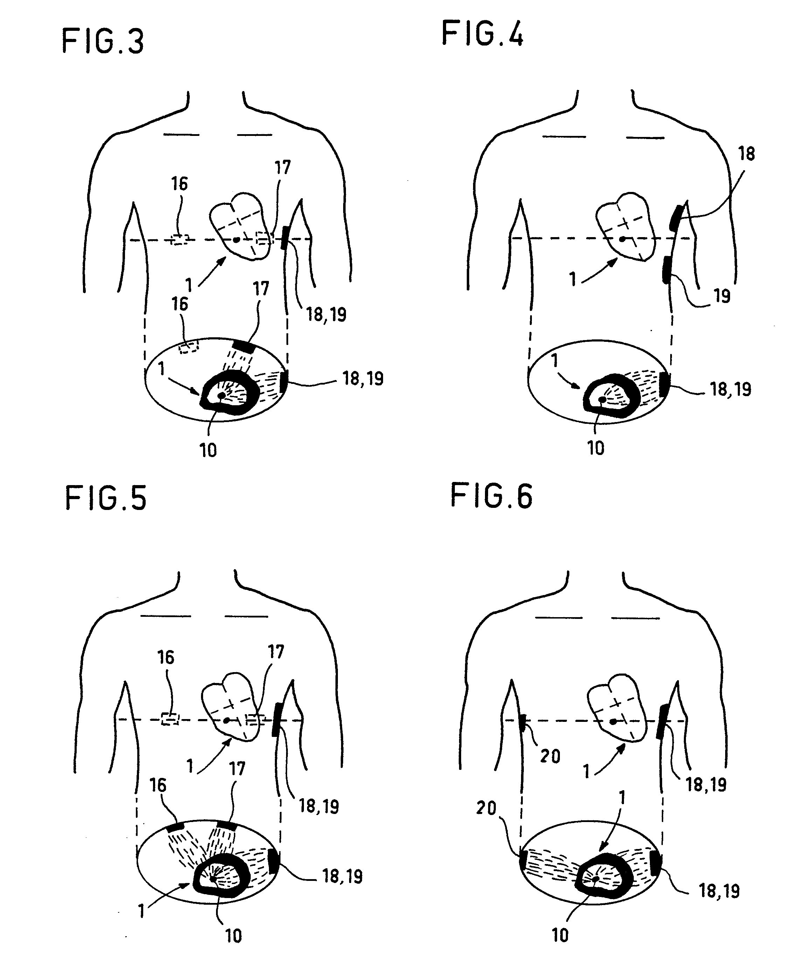

. la figure 3 est une vue analogue illustrant une autre variante de position des électrodes ;

. la figure 4 est une vue double frontale en transparence et en coupe selon un plan transversal passant par l'électrode endocavitaire avec figuration des emplacements des électrodes extérieures correspondant à une autre variante ;

. la figure 5 est une vue double analogue à celle de la figure 4 montrant une autre variante de position des électrodes extérieures ;

. la figure 6 est une vue analogue à celle de la figure 5 montrant une variante supplémentaire ;

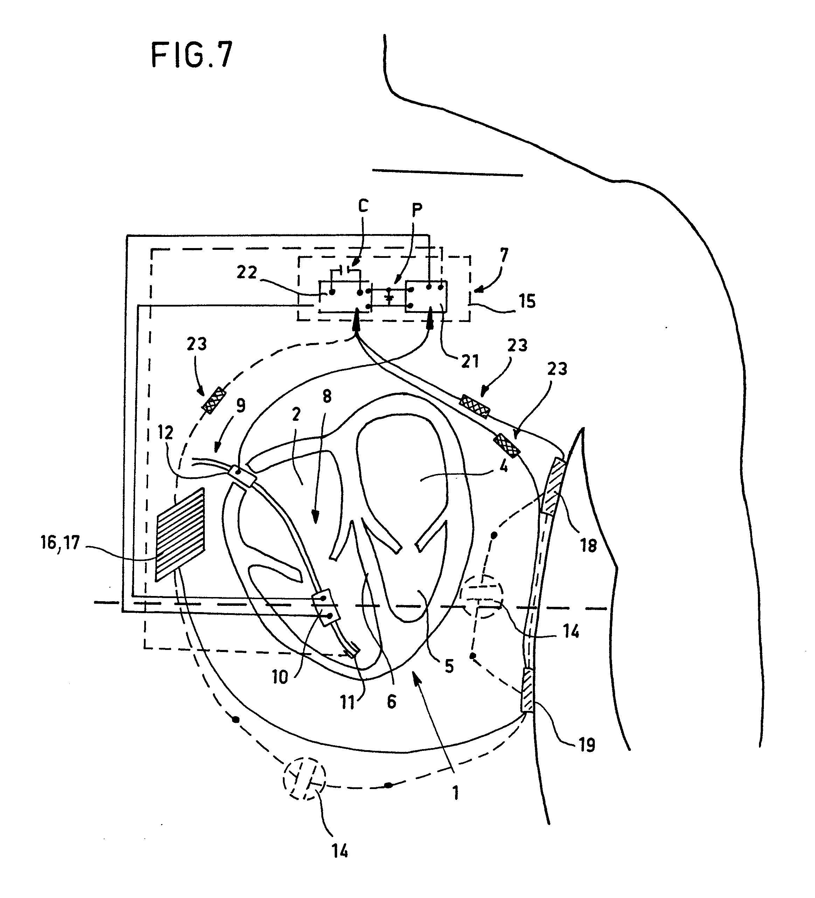

. la figure 7 est le schéma électrique des circuits de liaison entre les électrodes et les blocs des fonctions générales des circuits renfermés dans le boîtier d'alimentation et de commande ;

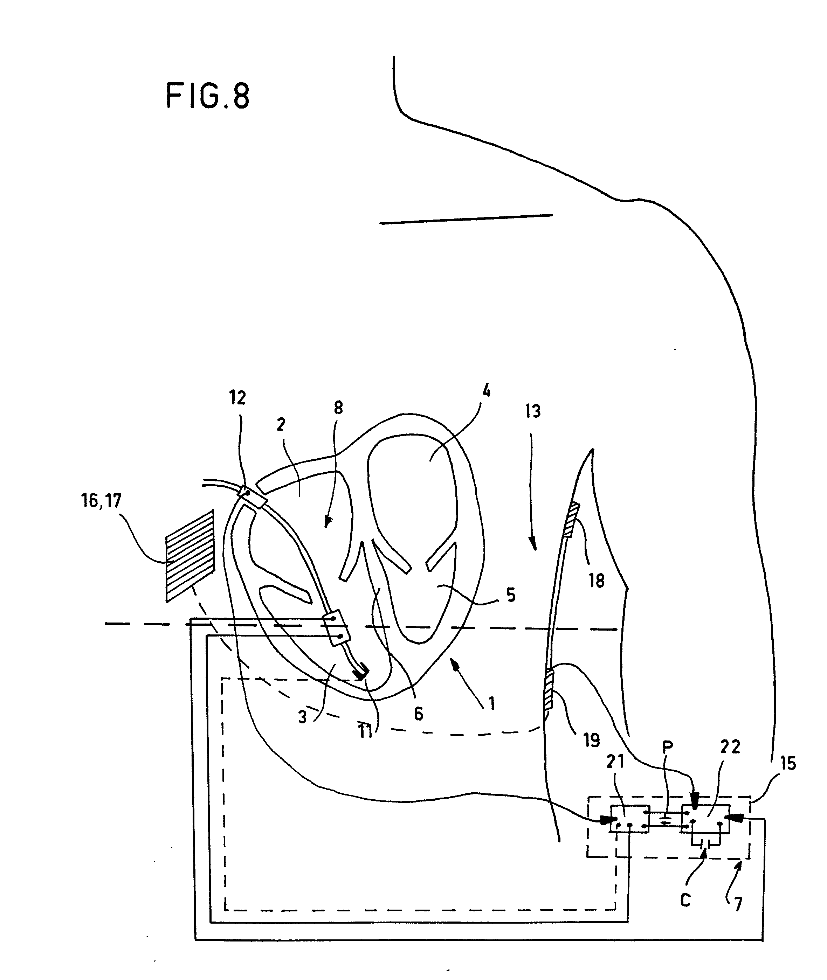

. la figure 8 est le schéma électrique d'une variante avec boîtier extérieur au corps et électrodes extérieures en contact avec la peau.

L'idée générale inventive consiste à prévoir un défibrillateur comprenant plusieurs électrodes dont le boîtier d'alimentation et de commande, électrodes implantables extérieurement au coeur ou en contact avec la peau et disposées selon des directions différentes de façon à permettre une répartition uniforme des lignes de courant distribuées en intensité pondérée selon des directions générales et élémentaires préférentiellement sur les zones et parois de plus grande épaisseur ou de plus grande masse musculaire au niveau des ventricules pour l'efficacité de propagation et d'excitation élémentaire individuelle des cellules et fibres musculaires, ces lignes de courant et directions participant efficacement à la défibrillation.

Une des caractéristiques de l'idée générale inventive consiste donc à créer des lignes efficaces de courant de plus grande intensité dans les régions du myocarde ventriculaire de plus forte épaisseur.

Ainsi, la présente invention est totalement indépendante du type de défibrillateur ou de défibrillateur-cardioverteur utilisé. Elle s'applique aussi bien aux défibrillateurs classiques qu'aux défibrillateurs simples ou multifonctions et notamment aux défibrillateurs à électrode de décharge maintenue écartée des parois par des moyens appropriés, en particulier maintenue en position centrale dans le ventricule droit.

Dans la description qui suit, les termes défibrillation et défibrillateur englobent la fonction de cardioversion et de l'appareil qui la met en oeuvre et le traitement concerne aussi bien la fibrillation ventriculaire que la tachycardie ventriculaire. Ainsi, le terme fibrillation couvre aussi le trouble de tachycardie ventriculaire.

L'invention s'applique aussi aux défibrillateurs à boîtier d'alimentation et de commande extérieur au corps ainsi qu'à des défibrillateurs à boîtier d'alimentation et de commande extérieur au corps utilisant plusieurs électrodes extérieures au corps c'est-à-dire en contact avec la peau ainsi que le montre la figure 8.

Le défibrillateur selon l'invention s'applique à un coeur 1 présentant une oreillette droite 2 et un ventricule droit 3 ainsi qu'une oreillette gauche 4 et un ventricule gauche 5 formés dans un muscle appelé myocarde. Les ventricules sont séparés par une paroi 6 appelée septum. Le myocarde est commandé de façon répétitive en mouvements de contraction et de relâchement susceptibles de présenter chez certains patients le risque d'un accès de fibrillation ventriculaire.

Cet état de fibrillation peut être réduit par un choc électrique consécutif à la décharge rapide et automatique d'un condensateur à travers le myocarde

Le défibrillateur selon l'invention vise au déclenchement d'un choc unique et efficace de défibrillation de faible énergie résultant de la décharge d'un condensateur C.

Un boîtier électronique 7 d'alimentation, de commande et de surveillance est relié à un ensemble 8 d'électrodes intérieures au coeur portées par un cathéter 9 introduit dans le coeur par la veine cave supérieure. Ledit cathéter porte au moins une électrode de décharge 10 située dans le ventricule droit 3, par exemple sous la forme d'une bague, et, le cas échéant, une électrode distale 11 ainsi qu'une troisième électrode 12 située dans l'oreillette droite 2 à l'embouchure de la veine cave.

Les électrodes intérieures sont reliées individuellement aux circuits concernés du boîtier 7 d'alimentation de commande et de surveillance que l'on décrira ci-après.

Le boîtier électronique 7 du défibrillateur selon l'invention est relié également à un ensemble 13 de plusieurs électrodes extérieures au coeur.

Ces électrodes extérieures au coeur sont de préférence implantées en position sous-cutanée au niveau des tissus musculaires ou graisseux sur le pourtour du thorax, mais peuvent tout aussi bien être extérieures au corps en contact avec la peau par l'intermédiaire d'un gel électriquement conducteur.

La liaison électrique des électrodes extérieures au coeur peut s'effectuer de différentes façons.

Tout d'abord, les électrodes sont reliées toutes individuellement au circuit correspondant du boîtier électronique 7. Selon une variante les électrodes sont reliées électriquement entre elles, l'une d'entre elles seulement est reliée au boîtier.

Une autre variante consiste à relier les électrodes entre elles par l'intermédiaire d'éclateurs tels que 14 constituant autant de courts-circuits pour les hautes tensions de décharge mais isolés en basse tension, éclateurs aux bornes desquels on peut prélever un signal électrophysiologique.

Les électrodes extérieures au coeur formant l'ensemble 13 présentent la particularité d'être distantes du coeur, c'est-à-dire suffisamment éloignées et étendues dans des directions différentes pour constituer une distribution préférentielle multidirectionelle des lignes efficaces de courant, de part et d'autre d'une ligne directe joignant l'électrode de décharge et l'électrode extérieure concernée.

Les lignes efficaces de courant forment ainsi un angle solide dont le sommet est constitué par l'électrode de décharge et dont l'ouverture varie en fonction de l'éloignement et de la surface de l'électrode extérieure correspondante.

On décrira ci-après les zones préférentielle d'emplacement des électrodes extérieures au coeur.

Préférentiellement, les électrodes sont disposées dans un plan transversal coupant le thorax à la hauteur des ventricules et/ou dans une zone définie par la ligne axillaire gauche.

Selon une variante, le boîtier électronique 7 présente un corps 15 rendu électriquement conducteur et utilisé comme électrode extérieure au coeur. Dans ce cas, il est placé de préférence en position abdominale ou sousclavière.

On décrira maintenant ci-après des positions générales dans les zones définies ci-dessus que peuvent occuper les électrodes extérieures au coeur ceci, en référence aux figures de 1 à 6.

On distingue ainsi des configurations à trois électrodes et des configurations à deux électrodes seulement. Elles se divisent en deux groupes : d'une part des électrodes axillaires telles que 16 ou 17 ou 16 et 17 en nombre optimal égal è deux disposées préférentiellement dans une zone ou sur la ligne axillaire gauche et d'autre part des électrodes dorsales 18 ou 19 ou 18 et 19 situées sur le pourtour du thorax en dehors de la zone axillaire.

De façon préférentielle, les électrodes dorsales sont situées dans le plan transversal coupant le coeur au niveau de l'électrode de décharge 10 placée dans le ventricule droit.

On examinera maintenant, en référence aux figures, quelconque cas particuliers de positions d'électrodes extérieures au coeur ayant conduit à des résultats remarquables.

Une des configurations caractéristiques concerne des électrodes extérieures au coeur contenues dans le plan transversal passant par l'électrode de décharge référencée 10, pour l'une dans la zone ou sur la ligne axillaire gauche et pour l'autre dans le dos sous l'une ou l'autre des omoplates (figure 3).

Une variante dérivée concerne le même type de disposition générale avec deux électrodes axillaires dans la zone ou sur la ligne axillaire gauche disposées de part et d'autre du plan transversal concerné (figures 1 et 2).

Une variante simplifiée de celle ci-dessus ne comporte que deux électrodes axillaires situées dans la zone ou sur la ligne axillaire gauche ( figure 4).

Une autre variante présente une seule électrode axillaire dans le plan transversal passant par l'électrode de décharge 10 et une ou deux électrode(s) dorsale(s) située(s) dans ledit plan transversal (figure 5).

Une des électrodes référencées 20 , de plus petite surface, est placée à l'intersection du plan transversal concerné et de la zone ou de la ligne axillaire droite. Il s'agit du cas particulier représenté sur la figure 6.

Elles présentent aussi la particularité d'être disposées de part et d'autre du coeur se partageant en électrodes extérieures latérales axillaires et en électrodes dorsales et plus particulièrement latérales dorsales de manière à obtenir une distribution préférentielle des lignes de courant de l'électrode de décharge à travers le septum et le ventricule gauche d'une part et à travers la paroi externe du ventricule droit d'autre part.

Pour des raisons chirurgicales, le nombre d'électrodes implantables doit être limité. Un nombre supérieur à six est déjà jugé excessif ici.

Ainsi, selon les variantes préférées, les électrodes extérieures sont en nombre compris entre deux et quatre, disposées latéralement au coeur au niveau des ventricules de part et d'autre du septum.

On comprend que le fractionnement de l'intensité dans plusieurs directions permet, comme indiqué ci-dessus, d'augmenter ou d'opérer à des niveaux d'intensité de décharge plus forts sans aucunement craindre la nocivité liée à l'augmentation de la densité de courant traversant les tissus.

Comme indiqué, on distingue deux groupes d'électrodes, les premières dites latérales axillaires et les secondes dites dorsales et plus particulièrement latérales dorsales situées, pour ces dernières, dans un plan transversal passant par les ventricules en regard de la paroi myocardique du ventricule droit.

Les électrodes extérieures au coeur se développent selon leur position et leur fonction sur des surfaces de l'ordre de quelques cm² à quelques dizaines de cm², de manière à ouvrir les angles solides de distribution des lignes efficaces de courant à partir de l'électrode de décharge et à travers le septum et le myocarde adjacent du ventricule droit ou gauche.

L'électrode ou les électrodes dorsale(s) est ou sont disposée(s) de manière à développer des lignes efficaces de courant latéralement vers l'arrière du coeur, à travers d'importants masses de myocarde.

A l'observation de la figure 1, on peut constater nettement la multiplication et la dispersion des lignes efficaces de courant à travers une grande zone du myocarde du ventricule gauche.

Dans cette région, le myocarde est plus épais et il y a lieu de multiplier les lignes de courant efficaces dans chaque angle solide partant de l'électrode de décharge vers chacune des électrodes extérieures latérales axillaires.

Le but visé est d'atteindre, en jouant sur ces paramètres, totalement une masse maximale de myocarde ventriculaire et plus particulièrement, par des intensités supérieures, les régions de plus forte épaisseur du muscle myocardique : ventricule gauche, septum, apex.

Les distributions des lignes efficaces de courant dépendent de la position et de la surface des électrodes. On pourra ainsi multiplier de façon plus importante les lignes de courant traversant le ventricule gauche par une augmentation appropriée de la surface de la ou des électrodes concernées.

Les lignes efficaces de courant vers le côté droit du coeur semblent devoir être moins intenses, en raison vraisemblablement de l'épaisseur plus faible du ventricule droit. Aussi, peut-on se contenter d'une électrode de plus faible surface à droite.

Selon diverses constatations, les effets indiqués ci-dessus sont obtenus aussi bien avec des électrodes sous-cutanées qu'avec des électrodes en contact avec la peau et avec un boîtier 7 situé en dehors du corps.

Le boîtier électronique 7 renferme les divers circuits de contrôle, de surveillance et de commande de la charge et de la décharge, ainsi que la ou les piles P et le condensateur C de défibrillation.

Comme indiqué, et selon un des avantages principaux de l'invention, la taille du condensateur est réduite en raison du bon rendement énergétique de défibrillation lié à ce type de défibrillateur.

Plus précisément, mais de façon non limitative, le boîtier électronique 7 renferme un circuit 21 de détection et de surveillance des battements cardiaques à partir d'un signal électrophysiologique prélevé, entre l'électrode distale et l'électrode de décharge par exemple, ou entre d'autres électrodes.

Il renferme aussi un circuit 33 de charge et de décharge du condensateur C formé essentiellement par un convertisseur haute tension alimenté à partir de la pile P et commandé par un circuit décisionnel comprenant une boucle de verrouillage empêchant automatiquement une deuxième charge après une première décharge en agissant sur l'interrupteur d'alimentation du circuit de charge.

Comme indiqué, selon une des variantes préférées, les moyens décrits ci-dessus s'appliquent à des défibrillateurs du type à électrode de décharge maintenue en position centrale dans le ventricule droit ou présentant des moyens pour la maintenir à distance des parois.

Ce maintien apporte une amélioration sensible dans l'efficacité de défibrillation pour une énergie donnée.

Le défibrillateur selon l'invention présente un taux d'efficacité élevé. Une seule décharge permet d'obtenir la défibrillation. Ainsi, il procède par un choc unique et non séquentiel comme dans d'autres défibrillateurs moins performants.

Pour ce faire, comme indiqué ci-dessus, le circuit de charge, comporte une boucle de verrouillage empêchant toute recharge automatique du condensateur après une première décharge.

Les liaisons électriques des électrodes extérieures au circuit correspondant du boîtier 7 présentant chacune un détecteur de courant de crête tel que 23 utilisé pour optimiser la configuration des électrodes et leurs surfaces respectives.

La présente invention se rapporte également à un procédé de test et d'évaluation de l'efficacité du défibrillateur comportant plusieurs électrodes extérieures reliées électriquement entre elles, selon lequel on effectue la mesure, au moment du choc, des courants de crête dans les différentes liaisons reliant les électrodes extérieures au circuit de décharge du boîtier d'alimentation et de commande à l'aide des éléments de mesure et de réglage, détecteurs ou autres, insérés dans les liaisons et de comparer ces courants entre eux de manière à contrôler et à modifier les électrodes ou les intensités du courant dans chacune des liaisons électriques individuelles des électrodes extérieures au boîtier.

L'invention se rapporte également à un procédé de test et d'évaluation de l'efficacité d'un défibrillateur comportant plusieurs électrodes extérieures selon lequel on applique des impulsions de stimulation entre l'électrode endocavitaire utilisée comme électrode de décharge et les électrodes extérieures reliées électriquement entre elles et on vérifie ou modifie les positions de ces électrodes ou les intensités du courant apporté à ces électrodes extérieures au coeur jusqu'à l'obtention d'un seuil de stimulation le plus élevé possible ou supérieur à une valeur prédéterminée.

L'invention a été décrite ci-dessus en détail. Il est bien entendu cependant que diverses modifications simples, adjonctions, variantes directes, substitutions par des moyens équivalents entrent dans le cadre de la présente protection.

## Revendications

1. Défibrillateur pour patients souffrant de troubles graves du rythme cardiaque, comportant au moins une électrode de décharge (10) intérieure au coeur, située dans le ventricule droit et au moins une électrode extérieure éloignée du coeur caractérisé en ce que les électrodes extérieures sont reliées électriquement entre elles formant un ensemble (13) relié électriquement au circuit correspondant d'un boîtier électronique (7) d'alimentation et de commande, électrodes extérieures disposées pour que la densité de courant à travers le myocarde ventriculaire se répartisse de façon homogène par une multitude de lignes de courant traversant préférentiellement les zones les plus épaisses du myocarde représentant l'essentiel de la masse myocardique en vue d'obtenir un seuil de défibrillation et/ou de cardioversion minimal et en ce que le choc de défibrillation est unique et à courant fort se départageant en plusieurs directions de courant.

2 Défibrillateur selon la revendication 1 caractérisé en ce que chaque liaison électrique individuelle des électrodes extérieures au circuit du boîtier électronique (7) d'alimentation et de commande comporte des détecteurs (23) permettant de mesurer le courant de crête passant par cette liaison.

3. Défibrillateur selon les revendications 1, et 2, caractérisé en ce que les électrodes implantables extérieurement au coeur sont placées en position sous-cutanée au niveau des tissus musculaires ou graisseux sur le pourtour du thorax.

4. Défibrillateur selon les revendications 1, 2, et 3, caractérisé en ce que les électrodes extérieures sont disposées dans un plan transversal coupant le thorax à hauteur des ventricules.

5. Défibrillateur selon la revendication 4 caractérisé en ce que le plan transversal coupe le thorax à la hauteur de l'électrode de décharge (10).

6. Défibrillateur selon la revendication 4, caractérisé en ce que les électrodes extérieures (18) et (19) sont disposées dans la zone ou sur la ligne axillaire gauche, la ou les électrodes dorsales étant disposées en regard du ventricule droit.

7. Défibrillateur selon la revendication 1, caractérisé en ce que le boîtier électronique (7) comporte un corps métallisé (15) implantable placé en position abdominale ou sous-claviaire utilisé comme l'une des électrodes extérieures au coeur.

8. Défibrillateur selon la revendication 6, caractérisé en ce qu'il comporte deux électrodes extérieures (18) et (19) placées dans la zone ou sur la ligne axillaire gauche, de part et d'autre d'un plan transversal passant à la hauteur de l'électrode de décharge (10) placée dans le ventricule droit.

9. Défibrillateur selon la revendication précédente caractérisé par une électrode supplémentaire est placée dans le dos.

10. Défibrillateur selon la revendication 1, caractérisé en ce qu'il comporte deux électrodes extérieures (16) et (17) placées dans le plan transversal passant à la hauteur de l'électrode (10) située dans le ventricule droit, l'une étant disposée à l'intersection de ce plan et de la ligne axillaire gauche et l'autre dans le dos, sous l'une ou l'autre des omoplates.

11. Défibrillateur selon les revendications de 1 à 4 caractérisé en ce que les électrodes extérieures au coeur sont extérieures au corps.

12 Procédé de test et d'évaluation d'un défibrillateur, caractérisé en ce que les courants de crête dans les différentes liaisons reliant les électrodes extérieures aux circuits du boîtier d'alimentation et de commande sont mesurés au moment du choc à l'aide des détecteurs (24) et comparés entre eux de manière à contrôler et à modifier les surfaces et les positions des électrodes dans le but d'obtenir un seuil de défibrillation minimal.

13. Procédé de test et d'évaluation d'un défibrillateur, caractérisé en ce que l'on applique des impulsions de stimulation entre l'électrode endocavitaire et les électrodes extérieures reliées électriquement entre elles et que l'on vérifie ou modifie la position des ces électrodes extérieures au coeur jusqu'à obtenir un seuil de stimulation le plus élevé possible ou supérieur à une valeur prédéterminée.

FIG.1

FIG.2

FIG.3

FIG.4

FIG.5

FIG.6

# FIG.7

# FIG.8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | GB-A-2 157 178 (ATESYS)<br>* Page 3, lignes 31-49; page 3, ligne 100 - page 4, ligne 89; page 4, ligne 121 - page 5, ligne 6; page 5, lignes 28-84; revendications 16,19; figures 1-3 *<br>--- | 1,3,7, 11 | A 61 N 1/39 |
| X | FR-A-2 257 312 (ZACOUTO)<br>* Page 15, ligne 40 - page 17, ligne 2; figures 9,10 * | 1,3,7, 11 | |
| A | | 4-6,8-10 | |
| | --- | | |
| X | GB-A-2 085 593 (HEWLETT-PACKARD)<br>* Page 3, ligne 1 - page 5, ligne 22; figures 1-3 *<br>--- | 12,13 | |
| A | US-A-3 886 950 (UKKESTAD)<br>* Colonne 3, ligne 51 - colonne 5, ligne 23; figures 1,2 *<br>--- | 2,12,13 | |
| A | DE-A-2 604 581 (OSYPKA)<br>* En entier *<br>--- | 12,13 | **DOMAINES TECHNIQUES RECHERCHES (Int. Cl.4)**<br><br>A 61 N |
| A | MEDICAL INSTRUMENTATION, vol. 20, no. 1, janvier-février 1986, pages 36-39, Arlington, US; M.J. KALLOK et al.: "Optimization of epicardial electrode size and implant site for reduced sequential pulse defibrillation thresholds"<br>* Pages 36-39 *<br>----- | 12,13 | |

Le présent rapport a été établi pour toutes les revendications

| Lieu de la recherche | Date d'achèvement de la recherche | Examinateur |
|---|---|---|
| LA HAYE | 20-09-1989 | LEMERCIER D.L.L. |